# EUROPÄISCHE PATENTSCHRIFT

(11) **EP 2 428 273 B1**
(45) Veröffentlichungstag und Bekanntmachung des Hinweises auf die Patenterteilung: **03.04.2013**
(21) Anmeldenummer: 11181109.7
(22) Anmeldetag: 13.09.2011
(51) Int. Cl.: B01L 7/02, B01L 9/06, B01J 19/00

(54) **Temperiervorrichtung zur thermischen Verfestigung von Wirkstoff-Beads**
Tempering device for thermal fastening of active agent beads
Dispositif de thermorégulation pour la fixation thermique de billes de matière active

(30) Priorität: 14.09.2010 DE 102010040685
(43) Veröffentlichungstag der Anmeldung: 14.03.2012
(73) Patentinhaber: Hamilton Bonaduz AG, 7402 Bonaduz (CH); Hamilton Company, Reno, Nevada 89502 (US)
(72) Erfinder: Etzold, Carsten, 7402 Bonaduz (CH); Neuhäusser-Wespy, Frieder, 8002 Zürich (CH); Schmökel, Mareen, 7028 Pagig (CH); Schmid, Claudio, 7188 Sedrun (CH); Seeber, Johann, 7000 Chur (CH)
(74) Vertreter: Trossin, Hans-Jürgen

(56) Entgegenhaltungen:
- WO-A2-2009/030908
- DE-A1- 19 849 714
- US-A1- 2003 190 260

## Beschreibung

Die vorliegende Erfindung betrifft eine Verwendung einer Temperiervorrichtung zum Temperieren wenigstens eines Probengefäßes, welche Temperiervorrichtung zur wahlweisen wärmeübertragenden Kopplung des Probengefäßes mit ihr und zur Trennung des Probengefäßes von ihr wenigstens eine sich längs einer Aufnahmeachse erstreckende Probengefäßaufnahme umfasst, wobei die Temperiervorrichtung eine erste Temperierzone und eine gesondert von dieser betreibbare zweite Temperierzone aufweist, wobei die erste und die zweite Temperierzone bezogen auf die Aufnahmeachse in unterschiedlichen axialen Bereichen der Probengefäßaufnahme angeordnet sind.

Derartige Temperiervorrichtungen sind als Laborgeräte weithin bekannt, etwa aus der WO 2009/030908 A2. Sie dienen in der Regel dazu, eine in dem Probengefäß aufgenommene Probe auf eine vorbestimmte Temperatur zu temperieren.

Sie werden in Fällen benutzt, in welchen es wünschenswert ist, das Probengefäß in unterschiedlichen Bereichen unterschiedlich zu temperieren, etwa weil die im Probengefäß aufgenommene Materie nur lokal auf eine gewünschte Temperatur gebracht werden soll, während in anderen Bereichen eine davon abweichende Temperatur gewünscht ist, oder weil in dem Probengefäß eine Flüssigkeit aufgenommen ist, in welche ein davon verschiedenes Material mit größerer Dichte als jener der aufgenommenen Flüssigkeit eingebracht werden soll und dieses Material beim Eintauchen in die und Absinken durch die temperierte Flüssigkeit unterschiedliche Temperaturzonen durchlaufen sollen.

Wirkstoff-Beads als Depot-Medikamente haben in der Medizin aufgrund der mit ihnen erzielten Behandlungserfolge enorme Bedeutung erlangt.

Wirkstoff-Beads umfassen in der Regel ein Trägermaterial, in welches ein Wirkstoff oder ein Material eingebettet sein kann, das aufgrund chemischer oder/und biologischer Reaktion einen Wirkstoff über eine endliche Wirkzeit hinweg erzeugt.

Da der Wirkstoff des Wirkstoff-Beads nach seiner Aufnahme im menschlichen oder tierischen Körper in der Regel eine Wirkung erzielt, sollen in der vorliegenden Anmeldung der Wirkstoff und das den Wirkstoff erzeugende Material mit dem Oberbegriff des biologisch aktiven Materials bezeichnet sein.

Als geeignetes Trägermaterial haben sich gelartige Materialien herausgestellt, von welchen Bio-Polymere, wie insbesondere Agarose, aufgrund ihrer guten Verträglichkeit im menschlichen oder tierischen Körper eine herausragende Stellung einnehmen.

Grundsätzlich liegen Trägermaterialien ursprünglich zur Einbettung des biologisch aktiven Materials darin als formlose fließfähige, jedoch verfestigbare Masse vor, in die das biologisch aktive Material eingemischt werden kann.

Im Laufe seiner Verfestigung nimmt der Wirkstoff-Bead eine, in der Regel kugelförmige, Gestalt an, wobei jedoch die Formstabilität des Wirkstoff-Beads je nach Verfestigungsfortschritt nicht besonders hoch ist und nicht mit einem starren Festkörper zu vergleichen ist.

Die niedrige Formstabilität während der Herstellungsphase macht den Wirkstoff-Bead überdies besonders sensibel für Kraftangriff von außen, was bisher die automatisierte Herstellung von Wirkstoff-Beads stark erschwert hat. Tatsächlich werden Wirkstoff-Beads für zahlreiche Anwendungsfälle nahezu vollständig von Hand hergestellt.

Aufgabe der vorliegenden Erfindung ist es daher, eine technische Lehre bereitzustellen, mit welcher es möglich ist, ein Probengefäß in verschiedenen Bereichen verschieden derart zu temperieren, dass mit der eingangs genannten Temperiervorrichtung eine Herstellung von Wirkstoff-Beads erleichtert ist.

Diese Aufgabe wird gelöst durch eine Verwendung einer eingangs genannten Temperiervorrichtung zur Erstarrung von Wirkstoff-Beads mit einem Trägermaterial, vorzugsweise einem gelartigen Trägermaterial, besonders bevorzugt einem Bio-Polymer, wie etwa Agarose, und mit einem in das Trägermaterial eingebetteten biologisch aktiven Material, wie etwa einem Wirkstoff oder/und einem Wirkstoff erzeugenden Material, in einem mit Fluid gefüllten und in der Probengefäßaufnahme aufgenommenen und durch die Temperiervorrichtung temperierten Probengefäß.

Bei dieser Verwendung kann vorgesehen sein, dass das Ausgangsmaterial des Wirkstoff-Beads, bevor es in das Fluid des Probengefäßes eingebracht wird, als im Wesentlichen formlose, fließfähige und verfestigbare Mischung, umfassend das Trägermaterial und das biologisch aktive Material, vorliegt. Mit "Ausgangsmaterial" ist dabei ein Material bezeichnet, welches wenigstens das Trägermaterial und das biologisch aktive Material bezeichnet.

Weiterhin kann das zuvor beschriebene temperierte Fluid auch bei einer anderen Art von Wirkstoff-Beads in vorteilhafter Weise zur Erstarrung dienen, nämlich bei solchen Wirkstoff-Beads, die als Bead-Rohling einen verfestigten Kern und eine nicht oder unvollständig verfestigte Umhüllung um den verfestigten Kern aufweisen. In diesem Fall kann das Eingeben eines derartigen Wirkstoff-Bead-Rohlings zur schonenden Verfestigung der Umhüllung dienen.

Insbesondere in dem bevorzugten Fall, dass mit der Temperiervorrichtung in einem Probengefäß eine stabile Schichtung einer Flüssigkeit, insbesondere Öl, mit längs der Aufnahmeachse unterschiedlich temperierten Temperierzonen erreicht werden soll, ist es vorteilhaft, wenn die Aufnahmeachse der wenigstens einen Probengefäßaufnahme in Schwerkraftwirkungsrichtung orientiert ist.

Eine derartige Schichtung einer temperierten Flüssigkeit, insbesondere eines temperierten Öls, kann für die Erzeugung von Wirkstoff-Beads von erheblichem Vorteil sein.

Derartige Bio-Polymere, insbesondere Agarose, sind thermisch verfestigbar, so dass in besonders vorteilhafter Weise zu verfestigendes Bio-Polymer schonend in die erste, wärmere Temperierzone einer durch die vorstehend beschriebene Temperiervorrichtung temperierte Flüssigkeit eingegeben wird, in Schwerkraftwirkungsrichtung absinkt, dabei in die kältere zweite Temperierzone derselben Flüssigkeit gelangt, wobei sich aufgrund der bei niedrigeren Flüssigkeitstemperaturen sich einstellenden höheren Viskosität die Sinkgeschwindigkeit verlangsamt und die Wärmeabgabe von dem Bio-Polymer zur Flüssigkeit in der zweiten Temperierzone sich aufgrund eben der niedrigeren Flüssigkeitstemperatur und damit des ursprünglich größeren Temperaturunterschieds zwischen Bio-Polymer und Flüssigkeit in der zweiten Temperierzone verstärkt.

Somit steht dem nun langsamer absinkenden Bead-Rohling bei gleicher Sinkstrecke eine größere Zeit zur Wärmeabgabe und damit zur Verfestigung zur Verfügung, so dass er ausreichend verfestigt ist, bevor er den Boden des Probengefäßes erreicht, wo er aufgrund seines Eigengewichts mechanisch belastet aufliegt.

Mit der eingangs genannten Temperiervorrichtung können wenigstens zwei Temperierzonen längs der Aufnahmeachse der Probengefäßaufnahme vorgesehen sein, welche gesondert betreibbar und damit auf unterschiedliche Temperaturen einstellbar sind.

Die Nennung einer ersten und einer zweiten Temperierzone soll nicht ausschließen, dass abgesehen von diesen beiden Temperierzonen weitere Temperierzonen vorgesehen sind, welche wiederum in von jenen der anderen Temperierzonen verschiedenen axialen Bereichen der Probengefäßaufnahme vorgesehen sind und welche weiterhin unabhängig von den jeweils anderen Temperierzonen auf eine vorbestimmte Temperatur einstellbar sind.

Durch die unabhängige Einstellbarkeit der ersten und der zweiten Temperierzone und gegebenenfalls weiterer Temperierzonen wird sichergestellt, dass diese auf unterschiedliche Temperaturen einstellbar sind.

Somit ist beispielsweise erreichbar, dass eine im Probengefäß aufgenommene und durch die vorliegende Temperiervorrichtung temperierte Flüssigkeit, welche eine von der Temperatur abhängige Viskosität aufweist, in den unterschiedlichen Temperierzonen eine unterschiedliche Viskosität aufweist.

Für die weitere Diskussion der Temperierzonen soll angenommen werden, dass die Probengefäßaufnahme der Temperiervorrichtung bei bestimmungsgemäßem Gebrauch mit einer Verlaufskomponente in Schwerkraftwirkungsrichtung verläuft und die erste Temperierzone bezogen auf die Schwerkraftwirkungsrichtung über der zweiten Temperierzone gelegen ist. Dies ist vor allen Dingen dann von Vorteil, wenn die erste Temperierzone dazu ausgebildet sein soll, ein in der Probenaufnahme aufgenommenes Probengefäß auf eine höhere Temperatur zu erwärmen als die zweite Temperierzone, da dann im Falle von im Probengefäß erwärmten Flüssigkeiten üblicherweise eine stabile Schichtung erhalten wird. Dies liegt daran, dass die Dichte von Flüssigkeiten gewöhnlich mit steigender Flüssigkeitstemperatur abnimmt.

Wenngleich ganz grundsätzlich die erste Temperierzone eine beliebige Temperatur in einem Probengefäß einstellen kann, das in der Probengefäßaufnahme aufgenommen ist, so ist dennoch bevorzugt, dass die erste Temperierzone dazu ausgebildet ist, ein in der Probengefäßaufnahme aufgenommenes Probengefäß auf eine Temperatur zu erwärmen, die gleich ist wie oder höher ist als die Umgebungstemperatur der Temperiervorrichtung. Vorzugsweise ist die erste Temperierzone dazu ausgebildet, ein in der Probengefäßaufnahme aufgenommenes Probengefäß auf eine Temperatur von 20 °C bis 30 °C, bevorzugt auf eine Temperatur von 20 °C bis 25 °C, besonders bevorzugt auf eine Temperatur von 22 °C bis 25 °C zu erwärmen.

Zusätzlich oder alternativ kann vorgesehen sein, dass die zweite Temperierzone dazu ausgebildet ist, ein in der Probengefäßaufnahme aufgenommenes Probengefäß auf eine Temperatur abzukühlen, die niedriger ist als die Umgebungstemperatur der Temperiervorrichtung. Vorzugsweise ist die zweite Temperierzone dazu ausgebildet, ein in der Probenaufnahme aufgenommenes Probengefäß auf eine Temperatur von 0 °C bis 15 °C, besonders bevorzugt auf eine Temperatur von 2,5 °C bis 12,5 °C zu erwärmen.

Zur möglichst einfachen Handhabung der Probengefäße und der Temperiervorrichtung bei der Bestückung der letzteren mit Probengefäßen ist es vorteilhaft, wenn die Probengefäßaufnahme eine Aufnahmeöffnung aufweist, durch welche hindurch das Probengefäß in die Probengefäßaufnahme einführbar und durch welchen hindurch das Probengefäß aus der Probengefäßaufnahme herausnehmbar ist.

Dann, wenn die Einführung des Probengefäßes durch die Aufnahmeöffnung hindurch in die Probengefäßaufnahme in Schwerkraftwirkungsrichtung geschieht, ist es zur Erzielung stabiler unterschiedlich temperierter Flüssigkeitszonen im Probengefäß vorteilhaft, wenn die erste Temperierzone näher an der Aufnahmeöffnung gelegen ist als die zweite Temperierzone.

Grundsätzlich kann die erste Temperierzone aufgrund einer beliebigen physikalischen Wechselwirkung Wärme zur Probengefäßaufnahme hin abgeben. Aus Gründen einer möglichst einfachen, aber genauen Temperatursteuerung ist allerdings bevorzugt, wenn in der ersten Temperierzone ein, vorzugsweise elektrisches, Heizelement vorgesehen ist. Zur Übertragung von Wärme von dem Heizelement zur Probengefäßaufnahme kann ein Wärmeübertragungsmedium vorgesehen sein. Hierbei ist ein starres Wärmeübertragungsmedium bevorzugt, wie etwa Metall, dessen elektrische Leitfähigkeit in weiten Bereichen mit der thermischen Leitfähigkeit korreliert, so dass ein Metall mit niedrigem spezifischem elektrischem Widerstand als Wärmeübertragungsmedium in der ersten Temperierzone bevorzugt ist.

Weiterhin verleiht die Verwendung von starren, also nicht flüssigen oder viskosen Wärmeübertragungsmedien der Temperiervorrichtung eine gewisse gewünschte Robustheit.

Hinsichtlich der zweiten Temperierzone ist es dagegen vorteilhaft, wenn diese durch ein zweites Wärmeübertragungsmedium durchströmbar ist. Mit strömbaren, also viskosen Wärmeübertragungsmedien können insbesondere die in der zweiten Temperierzone gewünschten tiefen Temperaturen leichter erzielt werden als durch entsprechende elektrische Elemente. Mit geeigneten viskosen Wärmeübertragungsmedien ist es überdies möglich, in der zweiten Temperierzone an der Probengefäßaufnahme Temperaturen von unter dem Gefrierpunkt von Wasser zu erreichen. Um sicher eine möglichst unabhängige Temperatureinstellung in den unterschiedlichen Temperierzonen zu gewährleisten, kann vorgesehen sein, dass zwischen der ersten und der zweiten Temperierzone eine Isolationszone zur thermischen Isolierung der ersten und der zweiten Zone voneinander vorgesehen ist.

Weiterhin ist vorteilhaft, wenn eine Temperiervorrichtung nicht nur eine einzige Probengefäßaufnahme aufweist und somit nur im Wesentlichen ein Probengefäß gleichzeitig temperieren kann, wenngleich dies von der vorliegenden Erfindung nicht ausgeschlossen sein soll. Vorteilhafter und wirtschaftlicher ist es jedoch, wenn die Temperiervorrichtung eine Mehrzahl von Probengefäßaufnahmen aufweist. In diesem Falle ist es zur Handhabung der Probengefäße und der Temperiervorrichtung weiter vorteilhaft, wenn die Mehrzahl von Probengefäßaufnahmen im Wesentlichen parallele Aufnahmeachsen aufweisen, so dass das Einführen und Entnehmen von Probengefäßen in die bzw. aus den Probengefäßaufnahmen über alle Probengefäßaufnahmen hinweg im Wesentlichen identisch ist.

Die vorliegende Erfindung wird nachfolgend anhand der beiliegenden Zeichnung näher erläutert. Es stellt dar:

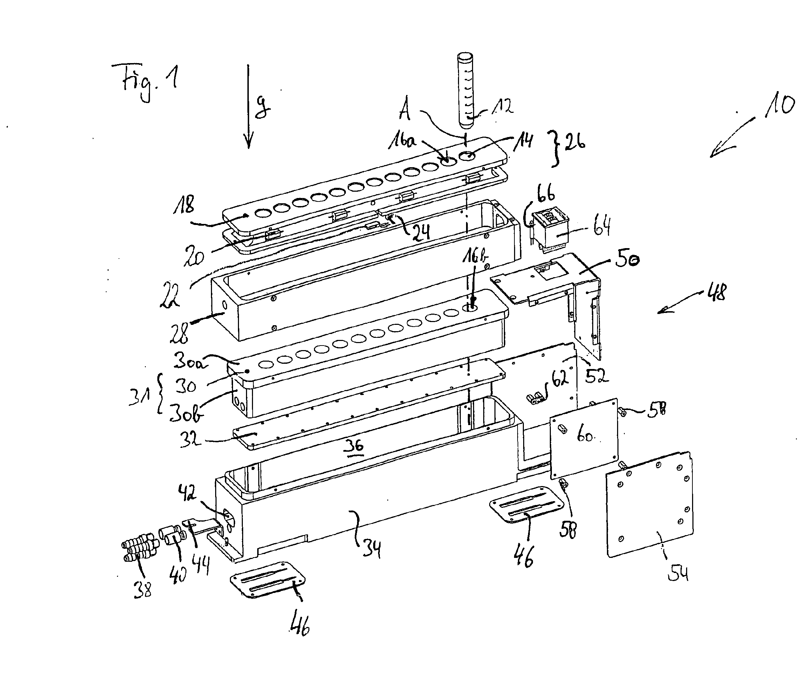

- Figur 1: eine Explosionsdarstellung einer Ausführungsform einer Temperiervorrichtung zur erfindungsgemäßen Verwendung der vorliegenden Anmeldung.

In Figur 1 ist eine Explosionsansicht einer Ausgestaltungsform einer Temperiervorrichtung für eine erfindungsgemäße Verwendung allgemein mit 10 bezeichnet.

Die Temperiervorrichtung 10 dient zur Temperierung von Probengefäßen 12, welche längs einer Aufnahmeachse A durch eine Aufnahmeöffnung 14 hindurch in eine Probengefäßaufnahme 16 im Inneren der Temperiervorrichtung 10 eingeführt und aus dieser entnommen werden können.

Die Heizvorrichtung 10 umfasst hierzu einen näher an der Aufnahmeöffnung 14 gelegenen Heizklotz 18, vorzugsweise aus Metall, welcher mit einer Mehrzahl, im dargestellten Beispiel 8, Heizwiderständen 20 bestückt ist. Die Heizwiderstände 20 werden durch einen Thermoschalter 22 auf Grundlage von Signalen eines Temperatursensors 24 gesteuert.

Der Heizklotz 18 bildet über seine axiale Erstreckung längs der Aufnahmeachse A eine erste Temperierzone 26.

Aufgenommen ist der Heizklotz 18 in einer Zwischenisolation 28, welche den Heizklotz 18 im montierten Zustand umgibt. In axialer Richtung folgt mit Abstand zu dem Heizklotz 18 ein Kühlklotz 30, welcher ebenfalls zur Bildung der Probengefäßaufnahme 16 beiträgt.

Genauer ist ein erster, in Fig. 1 oberer axialer Abschnitt 16a der Probengefäßaufnahme 16 im Heizklotz 18 ausgebildet und ist ein unterer Abschnitt 16b der Probengefäßaufnahme 16 im Kühlklotz 30 ausgebildet.

Der Kühlklotz 30 wird nach unten durch einen Boden 32 abgeschlossen, welcher an den Kühlklotz 30 angeschraubt oder sonstwie befestigt ist.

Der Kühlklotz, dessen Deckelabschnitt 30a im montierten Zustand der Temperiervorrichtung 10 von einem in Figur 1 unteren Bereich der Zwischenisolation 28 umgeben ist, ragt mit seinem Körperbereich 30b an welchen der Boden 32 angeschraubt ist, in eine Isolation 34 hinein.

Die Isolation 34 weist einen Hohlraum 36 auf, welcher größer als der darin einragende Körperabschnitt 30b des Kühlklotzes 30 ist, so dass der Kühlklotz 30 in der Isolation 34, genauer in dem verbleibenden Strömungsraum des Hohlraums 36, von Kühlflüssigkeit durchströmbar ist, welche durch Schlauchnippel 38 und Zwischenstücke 40 über eine Öffnung 42 in den Hohlraum 36 der Isolation 34 einleitbar ist.

Über ein Fixierblech 44 ist die Temperiervorrichtung 10 vorteilhafterweise in einem Untergrund fixierbar.

Alternativ oder zusätzlich kann die Temperiervorrichtung 10 über die Schlitz-Schiebeeinhängungen 46 an einem Untergrund befestigbar sein.

An dem vom Kühlmittelein- und ausgang gegenüberliegenden Längsende ist an der Temperiervorrichtung ein Steuergehäuse 48 vorgesehen, welches aus einem abgewinkelten Gehäuseteil 50 und zwei Seitenblechen 52 und 54 gebildet ist.

An eines der Seitenbleche, hier: Seitenblech 54, ist über Distanzstücke 58 eine Schaltungsplatine 60 angeordnet, auf welcher die Steuerungselektronik zur Temperatursteuerung der Temperaturzonen angeordnet ist. Im dargestellten Fall kann eine Stromzuführung zur Schaltungsplatine 60 über so genannte ,,Kabelbrides" 62 erfolgen, die vorzugsweise an dem Seitenblech 52 festgemacht sind, welches dem die Schaltungsplatine 60 aufnehmenden Seitenblech 54 entgegengesetzt ist.

Die Steuerschaltung der Schaltungsplatine 60 ist zur Einstellung der Temperaturen in den einzelnen Tempraturzonen vorzugsweise mit einem binär kodiertem dezimalen Schalter, einem so genannten BCD ("Binary Coded Decimal")-Schalter 64 verbunden.

Der Kühlblock 30 definiert eine zweite axiale Temperierzone 31.

Die Temperierzonen 26 und 31 sind mit axialem Abstand voneinander vorgesehen, grenzen daher nicht ummittelbar aneinander an und überlappen einander auch nicht.

Eine Leuchtdiode 66 oder ein anderes Signalmittel können vorgesehen sein, um die korrekte Funktion der Steuerung oder/und der Temperiervorrichtung 10 anzuzeigen.

Die in Figur 1 dargestellte Temperiervorrichtung 10 weist zwölf im Wesentlichen parallel angeordnete Probengefäßanordnungen 16 auf.

Selbstverständlich kann die Temperiervorrichtung auch nur eine Probengefäßaufnahme oder eine beliebige Mehrzahl von Probengefäßaufnahmen 16 aufweisen.

Vorzugsweise ist die Temperiervorrichtung 10 derart ausgebildet, dass in der ersten Temperierzone 26 eine Temperatur einstellbar ist, welche die Raumtemperatur umfasst oder/und höher als die Raum- bzw. Umgebungstemperatur der Temperiervorrichtung 10 ist.

Für die erfindungsgemäße Verwendung der Temperiervorrichtung 10 zur Temperierung von Öl in den Probengefäßen 12 zur Verfestigung der oben in der Beschreibungseinleitung genannten Wirkstoff-Beads, umfassend Bio-Polymer, vorzugsweise Agarose, wird der erste Temperierbereich 26 vorzugsweise auf Temperaturen von 20 °C bis 30 °C, besonders bevorzugt auf eine Temperatur von 20 °C bis 25 °C und in besonders starkem Maße bevorzugt auf eine Temperatur von 22 °C bis 25 °C eingestellt. Bei dieser Temperatur kann das zunächst als fließfähige Masse vorliegende Trägermaterial schonend, also ohne Temperaturschock, in das Öl des Probengefäßes eingegeben werden.

Die zweite Temperierzone 31, welche in Schwerkraftwirkungsrichtung g vorzugsweise axial unter der ersten Temperierzone liegt, können mit einer geeigneten Kühlflüssigkeit Temperaturen von unter dem Gefrierpunkt von Wasser, jedenfalls von unter der Raum- bzw. Umgebungstemperatur der Temperiervorrichtung 10 eingestellt werden.

Dies hat für die erfindungsgemäße Verwendung der vorliegenden Temperiervorrichtung 10 zur Herstellung von Wirkstoff-Beads den Vorteil, dass das üblicherweise in seiner Viskosität temperaturabhängige Öl im Probengefäß 12 in der zweiten, kühleren Temperierzone 31 zähflüssiger vorliegt, so dass ein Bead-Rohling, der in Schwerkraftwirkungsrichtung g im Probengefäß 12 absinkt, in seiner Sinkgeschwindigkeit verlangsamt wird und gleichzeitig in eine zunehmend kühlere Ölzone begibt, so dass ihm ausreichend Wärme entzogen werden kann, damit der Bead-Rohling ausreichend verfestigt am Grund des Probengefäßes 12 angelangt und durch den dort sich einstellenden Anlagedruck nicht beschädigt wird.

Abweichend von der vorliegenden Ausführungsform, die aufgrund ihres einfachen, aber zuverlässigen Aufbaus bevorzugt ist, kann die Temperiervorrichtung mehr als zwei Temperierzonen aufweisen, deren Temperaturen unabhängig voneinander einstellbar sind.

Dadurch, dass die erste Temperierzone im vorliegend beschriebenen Beispiel eine höhere Temperatur aufweist als die in Schwerkraftwirkungsrichtung g darunter liegende zweite Temperierzone, wird üblicherweise eine stabile Schichtung erhalten, da insbesondere Flüssigkeiten und Gase im Probengefäß üblicherweise eine mit steigender Temperatur abnehmende Dichte aufweisen.

## Patentansprüche

1. Verwendung einer Temperiervorrichtung (10) zur Erstarrung von Wirkstoff-Beads mit einem Trägermaterial, vorzugsweise einem gelartigen Trägermaterial, besonders bevorzugt einem Bio-Polymer, wie etwa Agarose, und mit einem in das Trägermaterial eingebetteten biologisch aktiven Material, wie etwa einem Wirkstoff oder/und einem Wirkstoff erzeugenden Material, in einem mit Fluid gefüllten und in einer Probengefäßaufnahme (16) aufgenommenen und durch die Temperiervorrichtung (10) temperierten Probengefäß (12), wobei genauer die Temperiervorrichtung zur wahlweisen wärmeübertragenden Kopplung des Probengefäßes (12) mit ihr und zur Trennung des Probengefäßes (12) von ihr wenigstens eine sich längs einer Aufnahmeachse (A) erstreckende Probengefäßaufnahme (16) umfasst, wobei die Temperiervorrichtung (10) eine erste Temperierzone (26) und eine gesondert von dieser betreibbare zweite Temperierzone (31) aufweist, wobei die erste und die zweite Temperierzone (26, 31) bezogen auf die Aufnahmeachse (A) in unterschiedlichen axialen Bereichen der Probengefäßaufnahme (16) angeordnet sind.

2. Verwendung nach Anspruch 1,
**dadurch gekennzeichnet, dass** die erste Temperierzone (26) der Temperiervorrichtung (10) dazu ausgebildet ist, ein in der Probengefäßaufnahme (16) aufgenommenes Probengefäß (12) auf eine Temperatur zu erwärmen, die gleich ist wie oder höher ist als die Umgebungstemperatur der Temperiervorrichtung.

3. Verwendung nach Anspruch 1 oder 2,
**dadurch gekennzeichnet, dass** die zweite Temperierzone (31) der Temperiervorrichtung (10) dazu ausgebildet ist, ein in der Probengefäßaufnahme (16) aufgenommenes Probengefäß (12) auf eine Temperatur abzukühlen, die niedriger ist als die Umgebungstemperatur der Temperiervorrichtung.

4. Verwendung nach einem der vorhergehenden Ansprüche,
**dadurch gekennzeichnet, dass** die Probengefäßaufnahme (16) eine Aufnahmeöffnung (14) aufweist, durch welche hindurch das Probengefäß (12) in die Probengefäßaufnahme (16) einführbar und durch welche hindurch das Probengefäß (12) aus der Probengefäßaufnahme (16) herausnehmbar ist, wobei die erste Temperierzone (26) näher an der Aufnahmeöffnung (14) gelegen ist als die zweite Temperierzone (31).

5. Verwendung nach einem der vorhergehenden Ansprüche,
**dadurch gekennzeichnet, dass** in der ersten Temperierzone (26) der Temperiervorrichtung (10) ein, vorzugsweise elektrisches, Heizelement (20) vorgesehen ist, welches Wärme über ein, vorzugsweise starres, erstes Wärmeübertragungsmedium zur Probengefäßaufnahme (16a) hin überträgt.

6. Verwendung nach einem der vorhergehenden Ansprüche,
**dadurch gekennzeichnet, dass** die zweite Temperierzone (31) der Temperiervorrichtung (10) durch ein zweites Wärmeübertragungsmedium durchströmbar ist.

7. Verwendung nach den Ansprüchen 5 und 6,
**dadurch gekennzeichnet, dass** zwischen der ersten (26) und der zweiten Temperierzone (31) der Temperiervorrichtung (10) eine Isolationszone zur thermischen Isolierung der ersten (26) und der zweiten Zone (31) voneinander vorgesehen ist.

8. Verwendung nach einem der vorhergehenden Ansprüche,
**dadurch gekennzeichnet, dass** die Temperiervorrichtung (10) eine Mehrzahl von Probengefäßaufnahmen (16) aufweist, welche vorzugsweise mit im Wesentlichen parallelen Aufnahmeachsen (A) vorgesehen sind.

9. Verwendung nach einem der vorhergehenden Ansprüche,
**dadurch gekennzeichnet, dass** die Aufnahmeachse (A) der wenigstens einen Probengefäßaufnahme (16) in Schwerkraftwirkungsrichtung (g) orientiert ist.

10. Verwendung nach einem der vorhergehenden Ansprüche,
**dadurch gekennzeichnet, dass** das Ausgangsmaterial des Wirkstoff-Beads, bevor es in das Fluid des Probengefäßes eingebracht wird, als im Wesentlichen formlose, fließfähige und verfestigbare Mischung, umfassend das Trägermaterial und das biologisch aktive Material, vorliegt.

11. Verwendung nach einem der vorhergehenden Ansprüche,
**dadurch gekennzeichnet, dass** der Wirkstoff-Bead, bevor er in das Fluid des Probengefäßes eingebracht wird, einen erstarrten Kern und eine den erstarrten Kern umgebende fließfähige Umhüllung, vorzugsweise umfassend das Trägermaterial, aufweist.

## Claims

1. Use of a temperature control device (10) to solidify active ingredient beads comprising a carrier material, preferably a gel-type carrier material, particularly preferably a biopolymer, such as agarose, and comprising a biologically active material embedded in the carrier material, such as an active ingredient and/or a material producing active ingredient, in a sample container (12) filled with fluid, accommodated in a sample container holder (16) and temperature-controlled by the temperature control device (10), wherein more specifically the temperature control device comprises, for optional heat-transferring coupling of the sample container (12) therewith and for separation of the sample container (12) therefrom, at least one sample container holder (16) extending along a holder axis (A), wherein the temperature control device (10) has a first temperature control zone (26) and a second temperature control zone (31) which can be operated separately therefrom, wherein the first and second temperature control zones (26, 31) are arranged in different axial regions of the sample container holder (16) relative to the holder axis (A).

2. Use according to claim 1, **characterised in that** the first temperature control zone (26) of the temperature control device (10) is designed to heat a sample container (12) accommodated in the sample container holder (16) to a temperature which is equal to or higher than the ambient temperature of the temperature control device.

3. Use according to either claim 1 or claim 2, **characterised in that** the second temperature control zone (31) of the temperature control device (10) is designed to cool a sample container (12) accommodated in the sample container holder (16) to a temperature which is lower than the ambient temperature of the temperature control device.

4. Use according to any of the preceding claims, **characterised in that** the sample container holder (16) has a holder opening (14) through which the sample container (12) can be introduced into the sample container holder (16) and through which the sample container (12) can be removed from the sample container holder (16), the first temperature control zone (26) being closer to the holder opening (14) than the second temperature control zone (31).

5. Use according to any of the preceding claims, **characterised in that** in the first temperature control zone (26) of the temperature control device (10) a preferably electrical heating element (20) is provided, which transfers heat to the sample container holder (16a) via a preferably rigid first heat transfer medium.

6. Use according to any of the preceding claims, **characterised in that** the second temperature control zone (31) of the temperature control device (10) may be flowed through by a second heat transfer medium.

7. Use according to claims 5 and 6, **characterised in that** an insulation zone is provided between the first (26) and second temperature control zones (31) of the temperature control device (10) to insulate the first (26) and second zones (31) thermally from one another.

8. Use according to any of the preceding claims, **characterised in that** the temperature control device (10) has a plurality of sample container holders (16), which are preferably provided with substantially parallel holder axes (A).

9. Use according to any of the preceding claims, **characterised in that** the holder axis (A) of the at least one sample container holder (16) is oriented in the direction of gravity (g).

10. Use according to any of the preceding claims, **characterised in that**, before it is introduced into the fluid of the sample container, the source material of the active ingredient bead is present as a substantially shapeless, flowable and solidifiable mixture, comprising the carrier material and the biologically active material.

11. Use according to any of the preceding claims, **characterised in that**, before it is introduced into the fluid of the sample container, the active ingredient bead comprises a solidified core and a flowable shell surrounding the solidified core, preferably comprising the carrier material.

## Revendications

1. Utilisation d'un dispositif de thermorégulation (10) pour la solidification de billes de matière active avec un matériau de support, de préférence un matériau de support de type gel, de préférence encore un bio-polymère, comme par exemple l'agarose, et avec un matériau biologiquement actif incorporé dans le matériau de support, comme par exemple une matière active et/ou un matériau produisant une matière active, dans un récipient d'échantillons (12) rempli de fluide et contenu dans un logement de récipient d'échantillons (16) et thermorégulé par le dispositif de thermorégulation (10), dans lequel plus exactement le dispositif de thermorégulation comprend, pour le couplage au choix avec transfert de chaleur du récipient d'échantillons (12) avec celui-ci et pour la séparation du récipient d'échantillons (12) de celui-ci, au moins un logement de récipient d'échantillons (16) s'étendant le long d'un axe de logement (A), dans laquelle le dispositif de thermorégulation (10) présente une première zone de thermorégulation (26) et une deuxième zone de thermorégulation (31) utilisable séparément de celle-ci, dans laquelle la première et la deuxième zones de thermorégulation (26, 31) sont disposées dans des régions axialement différentes du logement de récipient d'échantillons (16) par rapport à l'axe de logement (A).

2. Utilisation selon la revendication 1, **caractérisée en ce que** la première zone de thermorégulation (26) du dispositif de thermorégulation (10) est configurée de façon à chauffer un récipient d'échantillons (12) contenu dans le logement de récipient d'échantillons (16) à une température, qui est égale ou supérieure à la température ambiante du dispositif de thermorégulation.

3. Utilisation selon la revendication 1 ou 2, **caractérisée en ce que** la deuxième zone de thermorégulation (31) du dispositif de thermorégulation (10) est configurée de façon à refroidir un récipient d'échantillons (12) contenu dans le logement de récipient d'échantillons (16) à une température, qui est inférieure à la température ambiante du dispositif de thermorégulation.

4. Utilisation selon l'une quelconque des revendications précédentes, **caractérisée en ce que** le logement de récipient d'échantillons (16) présente une ouverture de logement (14), à travers laquelle le récipient d'échantillons (12) peut être introduit dans le logement de récipient d'échantillons (16) et à travers laquelle le récipient d'échantillons (12) peut être retiré hors du logement de récipient d'échantillons (16), dans laquelle la première zone de thermorégulation (26) est plus proche de l'ouverture de logement (14) que la deuxième zone de thermorégulation (31).

5. Utilisation selon l'une quelconque des revendications précédentes, **caractérisée en ce qu'**il est prévu, dans la première zone de thermorégulation (26) du dispositif de thermorégulation (10), un élément chauffant (20), de préférence électrique, qui transfère de la chaleur vers le logement de récipient d'échantillons (16a) au moyen d'un premier milieu de transfert de chaleur, de préférence rigide.

6. Utilisation selon l'une quelconque des revendications précédentes, **caractérisée en ce que** la deuxième zone de thermorégulation (31) du dispositif de thermorégulation (10) peut être parcourue par un deuxième milieu de transfert de chaleur.

7. Utilisation selon les revendications 5 et 6, **caractérisée en ce qu'**il est prévu, entre la première (26) et la deuxième (31) zones de thermorégulation du dispositif de thermorégulation (10), une zone d'isolation pour l'isolation thermique de la première (26) et de la deuxième (31) zones de thermorégulation l'une par rapport à l'autre.

8. Utilisation selon l'une quelconque des revendications précédentes, **caractérisée en ce que** le dispositif de thermorégulation (10) présente une pluralité de logements de récipient d'échantillons (16), qui sont prévus de préférence avec des axes de logement (A) essentiellement parallèles.

9. Utilisation selon l'une quelconque des revendications précédentes, **caractérisée en ce que** l'axe de logement (A) dudit au moins un logement de récipient d'échantillons (16) est orienté dans la direction de la force de gravité (g).

10. Utilisation selon l'une quelconque des revendications précédentes, **caractérisée en ce que** la matière première des billes de matière active, avant qu'elle soit introduite dans le fluide du récipient d'échantillons, se présente comme un mélange essentiellement sans forme, coulant et solidifiable, comprenant le matériau de support et la matière biologiquement active.

11. Utilisation selon l'une quelconque des revendications précédentes, **caractérisée en ce que** la bille de matière active, avant qu'elle soit introduite dans le fluide du récipient d'échantillons, présente un noyau solidifié et une enveloppe coulante entourant le noyau solidifié, comprenant de préférence le matériau de support.
